# EUROPEAN PATENT APPLICATION

(11) **EP 4 043 434 A1**
(43) Date of publication of application: **17.08.2022**
(21) Application number: 21156175.8
(22) Date of filing: 10.02.2021
(51) Int. Cl.: C07D 207/04, C07D 211/06, C07D 223/04, C07C 311/18, A61K 31/40, A61K 31/44, A61K 31/55, A61P 31/04, A61P 31/10

(54) **NOVEL SULFONYLIMINES**

(71) Applicant: Karl-Franzens-Universität Graz, 8010 Graz (AT)
(72) Inventor: SEEBACHER, Werner, 8044 Graz-Mariatrost (AT); HOFFELNER, Michael, 9545 Radenthein (AT)
(74) Representative: Pföstl, Andreas

(57) **Abstract**

The present invention relates to a process for preparing a sulfonylimine comprising a step of reacting a compound of general formula (II) 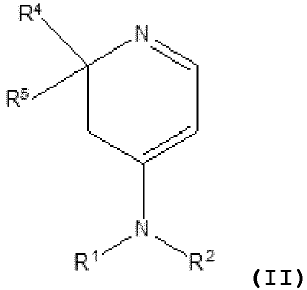 with a sulfonyl halide of general formula (III) 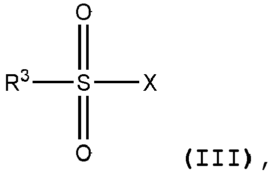 to obtain a sulfonylimine of general formula (I) 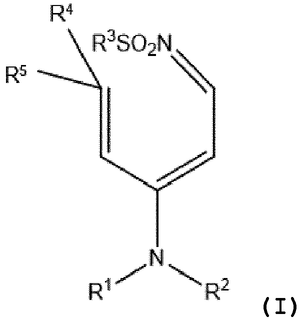 or a salt thereof,
as well as a sulfonylimine of general formula (I) or a salt thereof and its use as medicament.

## Description

### TECHNICAL FIELD

The present invention relates to novel sulfonylimine compounds, a process for preparing said compounds and the use of said compounds as medicament.

### BACKGROUND ART

Sulfonylimines are electron-rich compounds and as such valuable starting points (synthons) for cyclo-addition reactions. For example, they are used for preparing heterocycles such as e.g. functionalized tetrahydropyridines by cyclo-addition reactions (Gu, Z. et al. Chin. J. Chem. 2018, 36, 1130-1134) .

However, the available sulfonylimines and methods for preparing sulfonylimines are limited regarding the potential substitution patterns. Neidlein and Klotz describe the synthesis of electron-rich N-sulfonyl-1-azabutadiene substituted at C-2 and C-4 position and discussed that due to their diene character, these compounds may be suitable for cyclo-addition reactions (Neidlein, R. et al. Chem. Ber. 1985, 118, 3217-3226).

There is a need for new sulfonylimines and processes for preparing said compounds allowing for more structural variability in these valuable synthons for further reactions.

Moreover, certain sulfonylimines are described to exhibit agrochemical and pharmaceutical activities. Sulfonylimines have been investigated for their use in cancer therapy (Cardoso, J.M.S; et al. J Inorg Biochem, 2017, 166, 55-63) and in treating bacterial infections (Ewies, E.F; et al. J. Heterocyclic Chem., 2020, 57, 163-172). Sulfonylimines are also useful as agents for crop protection as pesticides (Okajima, N.; et al. Pestic. Sci., 1991, 32, 91-104) and as herbicides (EP 0 112 289).

It is therefore an object of the present invention to provide alternative processes for preparing new sulfonylimines, which are of high interest both as synthons for further chemical processing as well as due to their potential biological properties.

### SUMMARY OF THE INVENTION

The present invention provides a new sulfonylimine of general formula (I) or a salt thereof 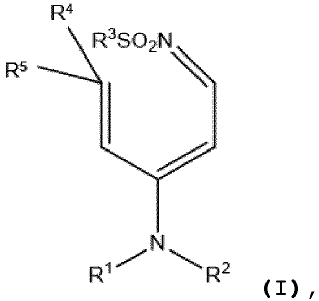 wherein
R¹ and R² are independently from each other selected from the group consisting of hydrogen and a substituted or unsubstituted C₁-C₁₀-alkyl group, or
R¹ and R² form together with the nitrogen to which they are bound a substituted or unsubstituted 5-, 6-, or 7-membered heterocyclyl group,
R³ is selected from the group consisting of
   a) a substituted or unsubstituted C₅-C₁₄-aryl group,
   b) a substituted or unsubstituted C₁-C₁₀-alkyl group,
   c) a substituted or unsubstituted C₅-C₁₄-aryl-C₁-C₄-alkyl group,
   d) a substituted or unsubstituted heteroaryl or a heteroaryl-C₁-C₄-alkyl group, wherein the heteroaryl group comprises a 5 to 14 membered aromatic ring system with at least one heteroatom selected from the group consisting of N, O, and S atoms,
   e) a substituted or unsubstituted heterocyclyl or a heterocyclyl-C₁-C₄-alkyl group, wherein the heterocyclyl group comprises a 5 to 14 membered non-aromatic ring system with at least one heteroatom selected from the group consisting of O and S atoms,
   f) a substituted or unsubstituted C₂-C₁₀-alkenyl group containing one or more double bonds,
   g) a substituted or unsubstituted C₂-C₁₀-alkinyl group containing one or more triple bonds,
   h) a substituted or unsubstituted C₅-C₁₄-aryl-C₂-C₄-alkenyl group containing one or more double bonds,
   i) a substituted or unsubstituted C₅-C₁₄-aryl-C₂-C₄-alkinyl group containing one or more triple bonds,
   j) a substituted or unsubstituted heteroaryl-C₂-C₄-alkenyl group containing one or more double bonds, and
   k) a substituted or unsubstituted heteroaryl-C₂-C₄-alkinyl group containing one or more triple bonds, wherein the heteroaryl is 5 to 14 membered aromatic ring system with at least one heteroatom selected from the group consisting of N, O, and S
   l) a substituted or unsubstituted heterocyclyl-C₂-C₄-alkenyl group containing one or more double bonds, and
   m) a substituted or unsubstituted heterocyclyl-C₂-C₄-alkinyl group containing one or more triple bonds, wherein the heterocyclyl is 5 to 14 membered non-aromatic ring system with at least one heteroatom selected from the group consisting of O and S,
and R⁴ and R⁵ both are independently from each other a substituted or unsubstituted C₁-C₁₀-alkyl group or R⁴ is a substituted or unsubstituted C₁-C₁₀-alkyl group and R⁵ is hydrogen.

It was surprisingly found that open-chain sulfonylimine compounds, i.e. sulfonylimines of general formula (I) or salts thereof, can be obtained upon reacting 2,3-dihydropyridin-4-amines, i.e. compounds of general formula (II), with sulfonyl halides, i.e. compounds of formula (III). The ring opening was unexpected in the light of the synthesis of cyclic carboxamides by reacting tetrahydropyridinylidene ammonium salts with benzoyl chlorides (Moshin N.; et al. Monatshefte für Chemie 2018, 149, 801-812). Surprisingly, the site-specific ring-opening reaction works with different substituents at the nitrogen atom in position 4 of the 2,3-dihydropyridin-4-amines and different sulfonyl halides yielding various sulfonylimines of general formula (I) (see examples 2 to 11).

Another aspect of the present invention relates to a process for preparing a sulfonylimine comprising a step of reacting a compound of general formula (II) 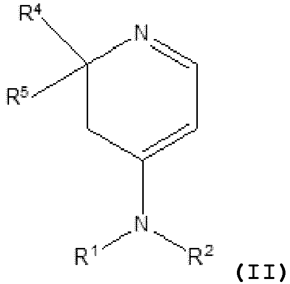 with a sulfonyl halide of general formula (III) 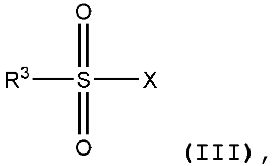 to obtain a sulfonylimine of general formula (I) or a salt thereof, 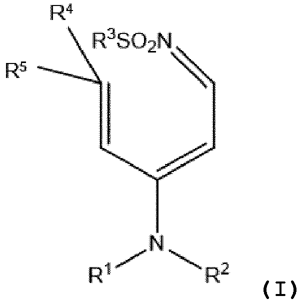 wherein
R¹ and R² are independently from each other selected from the group consisting of hydrogen and a substituted or unsubstituted C₁-C₁₀-alkyl group, or
R¹ and R² form together with the nitrogen to which they are bound a substituted or unsubstituted 5-, 6-, or 7-membered heterocyclyl group,
R³ is selected from the group consisting of
   a) a substituted or unsubstituted C₅-C₁₄-aryl group,
   b) a substituted or unsubstituted C₁-C₁₀-alkyl group,
   c) a substituted or unsubstituted C₅-C₁₄-aryl-C₁-C₄-alkyl group,
   d) a substituted or unsubstituted heteroaryl or a heteroaryl-C₁-C₄-alkyl group, wherein the heteroaryl group comprises a 5 to 14 membered aromatic ring system with at least one heteroatom selected from the group consisting of N, O, and S atoms,
   e) a substituted or unsubstituted heterocyclyl or a heterocyclyl-C₁-C₄-alkyl group, wherein the heterocyclyl group comprises a 5 to 14 membered non-aromatic ring system with at least one heteroatom selected from the group consisting of O and S atoms,
   f) a substituted or unsubstituted C₂-C₁₀-alkenyl group containing one or more double bonds,
   g) a substituted or unsubstituted C₂-C₁₀-alkinyl group containing one or more triple bonds,
   h) a substituted or unsubstituted C₅-C₁₄-aryl-C₂-C₄-alkenyl group containing one or more double bonds,
   i) a substituted or unsubstituted C₅-C₁₄-aryl-C₂-C₄-alkinyl group containing one or more triple bonds,
   j) a substituted or unsubstituted heteroaryl-C₂-C₄-alkenyl group containing one or more double bonds, and
   k) a substituted or unsubstituted heteroaryl-C₂-C₄-alkinyl group containing one or more triple bonds, wherein the heteroaryl is 5 to 14 membered aromatic ring system with at least one heteroatom selected from the group consisting of N, O, and S,
   l) a substituted or unsubstituted heterocyclyl-C₂-C₄-alkenyl group containing one or more double bonds, and
   m) a substituted or unsubstituted heterocyclyl-C₂-C₄-alkinyl group containing one or more triple bonds, wherein the heterocyclyl is 5 to 14 membered non-aromatic ring system with at least one heteroatom selected from the group consisting of O and S,
R⁴ and R⁵ both are independently from each other a substituted or unsubstituted C₁-C₁₀-alkyl group or R⁴ is a substituted or unsubstituted C₁-C₁₀-alkyl group and R⁵ is hydrogen,
and wherein X is a halide selected from the group consisting of F, Cl, and Br.

A further aspect of the present invention relates to the sulfonylimine of general formula (I) for use as a medicament, preferably for the use in the treatment or prevention of an infection, more preferably of a bacterial or fungal infection.

Disclosed is also a method of treating or preventing an infection, preferably a bacterial or fungal infection, wherein the method comprises the step of administering a sulfonylimine according to the present invention to a subject in need thereof.

### DESCRIPTION OF EMBODIMENTS

The compounds of general formula (II) which are used to synthesize the compounds of formula (I) can be manufactured according to the state of the art as described for example in Seebacher, W.; et al. (Monatshefte für Chemie 2015, 146, 1299-1308).

Preferably, the compound of general formula (II) and the respective sulfonylimine of general formula (I) are N,N-dialkylated. Thus, preferably, R¹ and R² both are independently from each other substituted or unsubstituted C₁-C₁₀-alkyl group, or R¹ and R² form together with the nitrogen to which they are bound a substituted or unsubstituted 5-, 6-, or 7-membered heterocyclyl group.

In an embodiment of the present invention, R₁ and R₂ form together with the nitrogen a non-aromatic heterocyclyl group. In particular, R₁ and R₂ form together with the nitrogen to which they a bound an aliphatic heterocyclyl group with 5 to 7 ring members, i.e. a substituted or unsubstituted 5-, 6-, or 7-membered heterocyclyl group.

For example, R¹ and R² form together with the nitrogen to which they are bound a heterocyclyl group selected from the group consisting of azepanyl, piperidinyl and pyrrolidinyl. Moreover, the heterocyclyl group can contain in addition to the nitrogen one or more heteroatoms selected from the group consisting of nitrogen, sulfur and oxygen, preferably sulphur and oxygen. The heterocyclyl group can be substituted by one or more substituent(s), preferably selected from the group consisting of amino, halide, cyano, hydroxyl, a C₁-C₄-alkoxy group, and a C₁-C₁₀-alkyl group, in particular amino and C₁-C₄-alkyl group.

In an embodiment of the present invention, wherein R¹ and R² both are a C₁-C₁₀-alkyl group, preferably R¹ and R² are independently from each other methyl or ethyl, more preferably methyl.

Preferably, both R¹ and R² are selected to result in a symmetric substitution of the nitrogen to which they are bound. Accordingly, in one embodiment of the present invention, R¹ and R² are identical to each other.

The compound of general formula (II) is preferably alkylated or dialkylated at position 2. The presence of alkyl groups at position 2 may have a beneficial influence on the ring opening upon reaction with the sulfonyl halides according to formula (III).

In a preferred embodiment of the present invention, both of R⁴ and R⁵ are a substituted or unsubstituted C₁-C₁₀-alkyl group, more preferably an unsubstituted C₁-C₆-alkyl group. More preferably, R⁴ and R⁵ both are methyl. In another embodiment, R⁴ is a substituted or unsubstituted C₁-C₁₀-alkyl group and R⁵ is hydrogen. If R⁵ is hydrogen, preferably, R⁴ is a C₃-C₆-alkyl group, in particular R⁴ is a branched C₃-C₆-alkyl group, such as for example isopropyl.

A sulfonyl halide of formula (III) as used in the process according to the invention can be synthesized according to the state of the art. Alkylsulfonyl chlorides are classically synthetized by a Reed reaction. Many alternative preparation methods for sulfonyl halides, such as for example the oxidation from disulfides (Prakash, G. K. Surya et al. J. Org. Chem., 2007, 72, 5847-5850), are available.

R³ is selected from the group consisting of the substituents a) to m). Optionally, the substituents a) to m) are substituted with one or more further substituent(s) selected from the group consisting of halides, cyano, hydroxyl, C₁-C₄-alkoxy group, and C₁-C₄-alkyl group.

The term "substituted" refers to a chemical entity being substituted with one or more substituents. The term "optionally substituted" relating to a chemical entity means that the chemical entity may be substituted or not, i.e. both situations are included. The term "one or more substituents" refers to from one to the maximum number of substituents possible, depending on the number of available substitution sites.

The term "C₅-C₁₄-aryl group" as used herein refers to an aromatic carbocyclic ring group having a specified number of carbon atoms. For example, C₅-C₆-aryl group refers to an aromatic ring having at least 5 and at most 6 carbon atoms. The term "aryl group" also refers to a multicyclic group having more than one aromatic ring, such as C₁₀-C₁₄-aryl group. Useful examples are cyclopentadienyl, phenyl, naphthyl, anthracyl and phenantryl. Typical substituted aryl groups are 4-chlorophenyl, 4-bromophenyl, 4-fluorophenyl, 4-trifluoromethylphenyl, 4-methylphenyl, 4-methoxyphenyl, 4-isopropylphenyl, 6-fluoro-naphthalen-1-yl, 6-chloro-naphtalen-1-yl, 6-methoxy-naphthalen-1-yl, 6-trifluoromethyl-naphthalen-1-yl, 3-methoxy-1-naphthalen-1-yl, 3-chloro-naphthalen-1-yl, 3-trifluoromethyl-naphthalen-1-yl, 3-fluoro-naphthalen-1-yl, 3,5-dichlorophenyl, 3,4-dichlorophenyl, 2-chlorophenyl, 2,6-dichlorophenyl, 3,5-difluoro-naphthalen-1-yl, 3,5-dichloro-naphthalen-1-yl, 3,5-dimethoxy-naphthalen-1-yl, 3,5-bis(trifluoromethyl)naphthalen-1-yl, 4-nitrophenyl, and 4-aminophenyl.

The term "C₁-C₁₀-alkyl group" as used herein refers to straight or branched hydrocarbon chain groups having a specified number of carbon atoms. For example, a C₁-C₁₀-alkyl group refers to a straight or branched alkyl group having at least 1 and at most 10 carbon atoms. Alkyl groups having a straight chain preferably represent C₁-C₇-alkyl. Useful branched alkyl groups, which preferably represent C₃-C₁₀-alkyl groups, are for example isopropyl, isobutyl, sec.-butyl, tert.-butyl, pentan-1-yl, pentan-2-yl, pentan-3-yl and 6-ethyloctan-2-yl.

The term "heteroaryl group" as used herein refers to an aromatic group having one or more heteroatoms (O, S or N). A multicyclic group having one or more heteroatoms, wherein at least one ring of the group is heteroaromatic, is referred to as a "heteroaryl group" as well. Typical substituted heteroaryl groups are 2-methylpyridin-4-yl, 3-methylpyridin-4-yl, 4-methylpyridin-3-yl, 5-methylpyridin-3-yl, 6-methylpyridin-3-yl, 2-methylpyridin-3-yl, 6-chloro-quinolin-1-yl, 6-fluoro-quinolin-1-yl, 6-methoxy-quinolin-1-yl, 6-trifluoromethyl-quinolin-1-yl, 7-chloro-quinolin-1-yl, 7-fluoro-quinolin-1-yl, 7-methoxy-quinolin-1-yl, 7-trifluoromethyl-quinolin-1-yl, 8-chloro-quinolin-1-yl, 8-fluoro-quinolin-1-yl, 8-methoxy-quinolin-1-yl, and 8-trifluoromethyl-quinolin-1-yl.

Useful aryl-alkyl groups, i.e. examples for a "C₅-C₁₄-aryl-C₁-C₄-alkyl group", wherein the aryl group preferably is a C₆-C₁₄-aryl group and the alkyl group is preferably a C₁-C₄-alkyl group, include for example benzyl, naphthalen-1-yl-methyl, naphthalen-2-yl-methyl, cyclopentadienyl-methyl, cycloheptadienyl-methyl, anthracene-methyl, and phenanthrene-methyl. Furthermore, phenylethyl, phenylpropyl and phenylbutyl groups are considered.

Useful heteroaryl-alkyl groups, i.e. examples for a "heteroaryl-C₁-C₄-alkyl group", wherein the heteroaryl group preferably is selected from a 5 to 10 membered heterocyclic ring and the alkyl group preferably represents C₁-C₄-alkyl, include for example pyridin-4-ylmethyl, pyridin-3-ylmethyl, pyridin-2-ylmethyl, pyridin-4-ylpropyl, pyridin-4-ylbutyl, pyrrol-2-ylmethyl, pyrrol-3-ylmethyl, indole-3-ylmethyl, indol-3-ylethyl and ferrocene-methyl.

For any aryl or heteroaryl group, C₆-C₁₀-aryl groups and 5 to 10 membered heteroaryl group, respectively, are preferred embodiments of the present invention. The aryl or heteroaryl group may be substituted. Preferred substituents are selected from the group consisting of halide, cyano, hydroxyl, nitro, amino, an alkyl group and an alkoxy group.

The term "heterocyclyl group" as used herein refers to a non-aromatic group having one or more heteroatoms (O or S). A multicyclic group having one or more heteroatoms, wherein at least one ring of the group is heterocyclic, is referred to as a "heterocyclyl group" as well. Typical heterocyclyl groups include for example tetraydrofuran, tetrahydrothiophene, 1,3-dioxolane, oxathiolane, tetrahydropyran, dioxane, thiane, dithiane, and trithiane.

Preferably, R³ is selected from a) and b), wherein optionally the substituents a) and b) are substituted by one or more substituent(s) selected from the group consisting of halide, cyano, hydroxyl, a C₁-C₄-alkoxy group, and a C₁-C₄-alkyl group.

In a preferred embodiment of the present invention, R³ is selected from the group of a substituted or unsubstituted C₆-C₁₄-aryl group and a substituted or unsubstituted C₁-C₁₀-alkyl group.

In one embodiment of the present invention, R³ is a C₁-C₁₀-alkyl group selected from the group consisting of methyl, ethyl, prop-1-yl, and prop-2-yl, i.e. R³ is a C₁-C₃-alkyl group. Preferably, R³ is methyl.

In another embodiment of the present invention, R³ is C₆-aryl, in particular C₆-aryl substituted in ring position 4, preferably selected from the group of 4-nitrophenyl, 4-chlorophenyl, 4-methylphenyl and phenyl.

The sulfonyl halide of general formula (III) comprises the halide X selected from the group consisting of F, Cl, and Br. In the process according to the invention, X is a leaving group. In a preferred embodiment of the present invention, X is Cl.

Depending on conditions during isolation of the sulfonylimine of general formula (I), the sulfonylimine of general formula (I) is obtained as free base or in the form of a salt, wherein the cation is the compound of formula (I) in a protonated form, e.g. a salt of formula (IV)

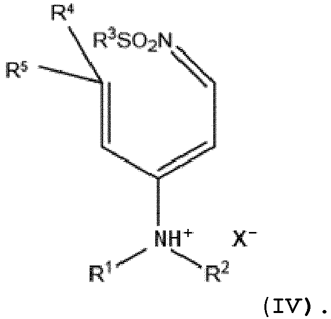

During the process according to the invention, the anion X⁻ may be introduced from the sulfonyl halide of formula (III). Accordingly, in one embodiment of the present invention, X⁻ is a halide anion selected from the group consisting of F⁻, Cl⁻, and Br⁻. However, depending on the conditions for isolating the sulfonylimine of formula (I), also other salts thereof can be obtained and, thus X⁻ may be an anion unrelated to the definition of X in formula (II).

For the sulfonylimine of formula (I) different stereochemical configurations are possible based on the E/Z-configuration at the double bonds. It is to be understood, that the drawing of the orientation in a formula should not exclude alternative configurations. Thus, in a compound according to formula (I) all configurational isomer should be understood to be covered if the stereochemistry is not explicitly indicated.

On the other hand, the products of the process according to the invention were obtained as isomeric pure compounds, for which it was found by NMR that the configuration of the double bonds between the carbon atoms corresponds to their orientation within the ring of the corresponding compound of formula (II). Accordingly, in a preferred embodiment of the present invention, the configuration of each of the C=C double bonds independently from each other is as visualized by the drawing of formula (I) and more preferably the configurations of both C=C double bonds are as visualized by the drawing of formula (I), i.e. in a configuration designated as E-configuration for the exemplified substitutions. The configuration at the double bond between the nitrogen of the sulfonamide structure and the adjacent carbon atom is expected to be readily invertable.

In one embodiment of the present invention, the sulfonylimine of formula (I) is selected from the group consisting of
(2E)-N-[5-methyl-3-(piperidin-1-yl)hexa-2,4-dien-1-ylidene]-4-nitrobenzenesulfonamide,
(2E)-N-[5-methyl-3-(pyrrolidin-1-yl)hexa-2,4-dien-1-ylidene]-4-nitrobenzenesulfonamide,
(2E)-N-[5-methyl-3-(piperidin-1-yl)hexa-2,4-dien-1-ylidene]benzenesulfonamide,
(2E)-4-chloro-N-[5-methyl-3-(piperidin-1-yl)hexa-2,4-dien-1-ylidene]benzenesulfonamide,
(2(E)-N-[5-methyl-3-(pyrrolidin-1-yl)hexa-2,4-dien-1-ylidene]benzenesulfonamide,
(2E)-N-(3-dimethylamino-5-methylhexa-2,4-dien-1-ylidene)benzenesulfonamide,
(2E)-4-methyl-N-[5-methyl-3-(pyrrolidin-1-yl)hexa-2,4-dien-1-ylidene]benzenesulfonamide,
(2E)-N-[5-methyl-3-(azepan-1-yl)hexa-2,4-dien-1-ylidene]benzenesulfonamide,
(2E)-N-[5-methyl-3-(piperidin-1-yl)hexa-2,4-dien-1-ylidene]methanesulfonamide,
(2E)-N-[3-(azepan-1-yl)-5-methylhexa-2,4-dien-1-ylidene]methanesulfonamide,
and salts thereof.

In the process according to the invention, the step of reacting the compound of general formula (II) with the sulfonyl halide of general formula (III) is preferably performed in an aprotic solvent, preferably an aprotic polar solvent. Suitable aprotic solvents are for example selected from the group consisting of dichloromethane, trichloromethan, carbontetrachloride, tetrachloroethan, tetrahydrofuran, and dioxane, preferably dichloromethane.

Preferably, for the step of reacting the compound of general formula (II) with the sulfonyl halide of general formula (III), the compound of general formula (II) and the sulfonyl halide of general formula (III) are provided in a molar ratio of about 1:1, e.g. 1:1.05 to 1:1.1.

In one embodiment of the present invention, the step of reacting the compound of general formula (II) with the sulfonyl halide of general formula (III) is performed in the presence of a molar excess of an organic base. In particular, the organic base is a trialkylamine, such as trimethylamine. In the presence of a base, the sulfonylimine of general formula (I) can be obtained as free base, whereas otherwise, the reaction typically leads to salts thereof.

The ring opening reaction does not require specific physical conditions, i.e. heat or pressure. Accordingly, the process according to the invention is preferably performed at room temperature or with initial cooling and under normal pressure.

In a preferred embodiment of the present invention, the step of reacting the compound of general formula (II) with the sulfonyl halide of general formula (III) is performed in the absence of water and/or oxygen, i.e. preferably under inert gas atmosphere. For example, the reaction mixture is put under argon atmosphere for the reaction time.

Typically, the reaction requires more than 24 h to be completed. Accordingly, the step of reacting the compound of general formula (II) with the sulfonyl halide of general formula (III) is completed over night or preferably within several days, e.g. 2, 3, 4, or 5 days.

The process according to the invention may further comprise subsequent steps of purification such as filtration, liquid-liquid separation, drying, column chromatography, and crystallization.

In one embodiment of the present invention, the process according to the invention further comprises one or more steps of (re)crystallizing the sulfonylimine of general formula (I) to obtain the sulfonylimine or a salt thereof in a crystalline form. Suitable solvents for (re)crystallization are for example ethyl acetate, diethyl ether, cyclohexane, ethanol or mixtures thereof. Accordingly, in one embodiment of the present invention, the present invention also relates to the sulfonylimine of general formula (I) or a salt thereof in crystalline form, in particular, in particular the chloride salts thereof in crystalline form.

It was further found that sulfonylimines of general formula (I) show antimicrobial activity, in particular antibacterial and antifungal activity. On the other hand, the cytotoxicity of the compounds towards mammalian cells, preferably human cells, was shown to be low. Hence, the compounds of the present invention can be administered safely to mammalians and humans.

Accordingly, in one embodiment of the present invention, the invention relates to the use sulfonylimines of general formula (I) in the treatment or prevention of an infection (i.e. a disease mediated by an infectious pathogen), preferably for use in treatment and prevention of a fungal or bacterial infection. In particular, the infection is a candidiasis.

In a preferred embodiment of the present invention, the sulfonylimines of general formula (I) for use as a medicament is administered to a subject in need thereof. Preferably, the subject is a human or an animal.

The present invention is further illustrated by the following examples, yet without being restricted thereto.

### EXAMPLES

The following abbreviations are used:
- CC: column chromatography
- CH: cyclohexane
- DMSO: dimethyl sulfoxide
- EtOAc: ethyl acetate
- EtOEt: diethylether
- EtOH: ethanol
- IC₅₀: half maximal inhibitory concentration
- MeOH: methanol
- NMR: nuclear magnetic resonance spectroscopy
- Rf: retention factor

### Example 1: Preparation of 2,2-dimethyl-2,3-dihydropyridin-4-amines

The 2,2-dimethyl-2,3-dihydropyridin-4-amines are educts of the reactions in Examples 2-11. They were prepared via a selective hydrogenation of their methylthio compounds as reported (Seebacher, W. et al. Monatshefte für Chemie, 2015, 146, 1299-1308; DOI:10.1007/s00706-015-1503-y).

The compounds were yielded as salts and before the reactions, the bases had to be set free:
The salts were stirred with a 2N aqueous NaOH solution for 30 min and then extracted exhaustively with chloroform dichloromethane or diethyl ether. The organic layers were combined and shaken with 2 N NaOH, dried over sodium sulfate, filtered and the solvent evaporated in vacuo yielding the free bases as yellowish or greenish oils. In this way, the following compounds were obtained:
- N,N,2,2-tetramethyl-2,3-dihydropyridin-4-amine,
- 2,2-dimethyl-4-(pyrrolidin-1-yl)-2,3-dihydropyridine,
- 2,2-dimethyl-4-(piperidin-1-yl)-2,3-dihydropyridine and
- 1-(2,2-dimethyl-2,3-dihydropyridin-4-yl)azepane.

### Example 2: (2E)-N-{5-Methyl-1-[(4-nitrophenyl)sulfonylimino] hexa-2,4-dien-3-yl}piperidin-1-ium chloride

589 mg (3.06 mmol) 2,2-dimethyl-4-(piperidin-1-yl)-2,3-dihydropyridine were co-distilled twice with dry benzene and dissolved in 30 ml of dry CH₂Cl₂. To this yellow solution 712 mg (3.21 mmol) of 4-nitrobenzene sulfonyl chloride were added. The reaction mixture was stirred for 5 days at room temperature under an Argon-atmosphere. During the reaction a white solid precipitated. Ethyl acetate was added with stirring and cooling until the crystallization seemed to be complete. The precipitate was filtered with suction, washed with pure ethyl acetate and dried *in vacuo* giving 433 mg of a yellow solid which is a mixture of 2 substances. The mixture was purified by means of CC with CH₂Cl₂ : MeOH = 9 : 1 (Rf: 0.84) as eluent giving 262 mg (0.63 mmol; 21 %) of N-{5-Methyl-1-[(4-nitrophenyl)sulfonylimino]hexa-2,4-dien-3-yl}piperidin-1-ium chloride as a yellow solid. For analytical purposes it was dissolved in CHCl₃, filtered, evaporated *in vacuo* and recrystallized from EtOH giving yellow needles.
m.p.: (EtOH): 183°C
¹H NMR (CDCl₃, δ, 400 MHz): 1.50 - 1.74 (m, 6H, 3CH₂), 1.66 (s, 3H, 6*-H), 1.99 (s, 3H, CH₃*), 3.50 - 3.53 (m, 4H, 2NCH₂), 5.67 (d, J = 11.0 Hz, 1H, 2-H), 5.73 (s, 1H, 4-H), 8.05 (d, J = 9.2 Hz, 2H, ArH), 8.28 (d, J = 8.8 Hz, 2H, ArH), 8.41 (d, J = 11.0 Hz, 1H, 1-H) ppm. ¹³C NMR (CDCl₃, δ, 100 MHz): 20.38 (C-6*), 23.94 (CH₂), 25.43 (CH₃*), 25.50, 26.76 (2CH₂), 48.36, 50.88 (2NCH₂), 97.86 (C-2), 116.49 (C-4), 123.83, 127.82 (ArC), 145.72 (C-5), 148.38, 149.19 (ArC_{q}), 166.66 (C-3), 169.65 (C-1) ppm.

### Example 3: (2E)-N-{5-Methyl-1-[(4-nitrophenyl)sulfonylimino] hexa-2,4-dien-3-yl}pyrrolidine-1-ium chloride

547 mg (3.07 mmol) of 2,2-dimethyl-4-(pyrrolidin-1-yl)-2,3-dihydropyridine were co-distilled twice with dry benzene and dissolved in 30 ml of dry CH₂Cl₂. To this yellow solution 714 mg (3.22 mmol) of 4-nitrobenzene sulfonyl chloride were added. The reaction mixture was stirred for 4 days at room temperature under an Argon-atmosphere. Ethyl acetate was added with stirring until the crystallization seemed to be complete. The precipitate was filtered with suction, washed with ethyl acetate and dried *in vacuo* giving 288 mg of a yellow up to brown solid. The crude product was purified by means of CC with CH₂Cl₂ : MeOH = 9 : 1 (Rf: 0.87) as eluent giving 50 mg (0.13 mmol; 4 %) of N-(2E)-{5-Methyl-1-[(4-nitrophenyl)sulfonylimino]hexa-2,4-dien-3-yl}pyrrolidine-1-ium chloride as a yellow solid. For analytical purposes it was dissolved in CHCl₃, filtered, evaporated *in vacuo* and recrystallized from EtOH giving fine yellow needles.
m.p.: (EtOH): 192°C
¹H NMR (CDCl₃, δ, 400 MHz) : 1.68 (s, 3H, 6*-H), 1.97 (br, s, 5H, CH₂, CH₃*), 2.04 - 2.08 (m, 2H, CH₂), 3.38 - 3.59 (m, 4H, 2NCH₂), 5.46 (d, *J* = 11.0 Hz, 1H, 2-H), 5.78 (s, 1H, 4-H), 8.05 (d, *J* = 8.8 Hz, 2H, ArH), 8.27 (d, *J* = 8.8 Hz, 2H, ArH), 8.37 (d, *J* = 11.0 Hz, 1H, 1-H) ppm. ¹³C NMR (CDCl₃, δ, 100 MHz) : 20.47 (C-6*), 24.76 (CH₂), 25.03 (CH₃*), 25.53 (CH₂), 48.84, 50.35 (2NCH₂), 98.79 (C-2), 116.98 (C-4), 123.86, 127.85 (ArC), 144.94 (C-5), 148.46, 149.22 (ArC_{q}), 165.63 (C-3), 168.25 (C-1) ppm.

### Example 4: (2E)-N-[5-Methyl-1-(phenylsulfonylimino)hexa-2,4-dien-3-yl]piperidin-1-ium chloride

589 mg (3.06 mmol) of 2,2-dimethyl-4-(piperidin-1-yl)-2,3-dihydropyridine were co-distilled twice with dry benzene and dissolved in 30 ml of dry CH₂Cl₂. To this yellow solution 579 mg (0.42 ml, 3.28 mmol) of benzene sulfonyl chloride were added. The reaction mixture was stirred for 5 days at room temperature under an Argon-atmosphere. The solvent was evaporated *in vacuo* and the residue (1370 mg, orange up to brown resin) was purified by means of CC with CH₂Cl₂ : MeOH = 20 : 1 and subsequently CH₂Cl₂ : MeOH = 9 : 1 (Rf: 0.78) as eluent. The fractions containing the pure product were collected (65 mg, orange resin) and crystallized from EtOH/EtOEt giving 31 mg of orange needles. Fractions containing the desired product and impurities (384 mg) were purified again by means of CC with CH₂Cl₂ : MeOH = 9 : 1 (Rf: 0.78) as eluent giving 101 mg of a yellow resin which was crystallized from EtOH/EtOEt and subsequently with EtOH giving 35 mg as yellow up to slightly orange needles. All in all 66 mg (0.18 mmol; 6 %) of N-(2E)-[5-Methyl-1-(phenylsulfonylimino)hexa-2,4-dien-3-yl]piperidin-1-ium chloride were obtained.
m.p.: (EtOH): 118°C
¹H NMR (CDCl₃, δ, 400 MHz) : 1.46 - 1.73 (m, 6H, 3CH₂) 1.62 (s, 3H, 6*-H) , 1.95 (s, 3H, CH₃*), 3.47 (br, s, 4H, 2NCH₂), 5.61 (d, *J* = 10.8 Hz, 1H, 2-H), 5.70 (s, 1H, 4-H), 7.41 - 7.48 (m, 3H, ArH), 7.88 (dd, *J* = 7.9, 1.7 Hz, 2H, ArH), 8.43 (d, *J* = 10.8 Hz, 1H, 1-H) ppm. ¹³C NMR (CDCl₃, δ, 100 MHz): 20.30 (C-6*), 24.10 (CH₂), 25.42 (CH₂, CH₃*), 26.70 (CH₂), 48.02, 50.50 (2NCH₂), 97.37 (C-2), 116.86 (C-4), 126.74, 128.58, 131.52 (ArC), 142.08 (ArC_{q}), 145.16 (C-5), 165.55 (C-3), 169.57 (C-1) ppm.

### Example 5: (2E)-4-Chloro-N-[5-methyl-3-(piperidin-1-yl)hexa-2,4-dien-1-ylidene]benzenesulfonamide

1 g (5.20 mmol) of 2,2-dimethyl-4-(piperidin-1-yl)-2,3-dihydropyridine were co-distilled twice with dry benzene and dissolved in 51 ml of dry CH₂Cl₂. To this solution 5.262 g (52.00 mmol, 7.25 ml) of dry triethylamine and 1.152 g (5.46 mmol) of 4-chloro benzene sulfonyl chloride were added. The reaction mixture was put under an Argon-atmosphere and was stirred for 2 days at room temperature. Water was added and stirring was continued for 15 minutes. The aqueous layer was extracted 5x with CH₂Cl₂. The combined organic layers were dried over anhydrous Na₂SO₄ and filtered. The solvent was evaporated *in vacuo* and the crude product (1.806 g, orange resin) was purified by means of CC with CH₂Cl₂ : MeOH = 30 : 1 (Rf: 0.35) as eluent. Fractions containing the product (1.298 g, brown up to orange resin) were collected and crystallized twofold with EtOAc /CH giving 306 mg of a white, cotton like solid which was recrystallized from EtOH giving 220 mg (0.60 mmol; 12 %) of (2E)-4-chloro-N-[5-methyl-3-(piperidin-1-yl)hexa-2,4-dien-1-ylidene]benzenesulfonamide as off white needles.
m.p.: 136°C (EtOH)
¹H NMR (DMSO-d₆, δ, 400 MHz): 1.36 - 1.64 (m, 6H, 3CH₂) 1.49 (s, 3H, 6*-H), 1.89 (s, 3H, CH₃*), 3.45 - 3.58 (m, 4H, 2NCH₂), 5.63 (d, *J* = 11.0 Hz, 1H, 2-H), 5.87 (s, 1H, 4-H), 7.58 (d, *J* = 8.7 Hz, 2H, ArH), 7.68 (d, *J* = 8.4 Hz, 2H, ArH), 8.13 (d, *J* = 11.1 Hz, 1H, 1-H) ppm. ¹³C NMR (DMSO-d₆, δ, 100 MHz): 20.15 (C-6*), 23.71 (CH₂), 24.94 (CH₃*), 25.63, 26.65 (2CH₂), 47.97, 50.65 (2NCH₂), 96.49 (C-2), 117.25 (C-4), 128.23, 129.26 (ArC), 136.60, 141.72 (ArC_{q}), 143.94 (C-5), 166.45 (C-3), 168.74 (C-1) ppm.

### Example 6: 2(E)-N-[5-Methyl-3-(pyrrolidin-1-yl)hexa-2,4-dien-1-ylidene]benzenesulfonamide

553 mg (3.10 mmol) of 2,2-dimethyl-4-(pyrrolidin-1-yl)-2,3-dihydropyridine were co-distilled twice with dry benzene and dissolved in 30 ml of dry CH₂Cl₂. To this solution 628 mg (6.20 mmol, 0.87 ml) of dry triethylamine and 548 mg (3.10 mmol, 0.40 ml) of benzene sulfonyl chloride were added. The reaction mixture was put under an Argon-atmosphere and was stirred for 2 days at room temperature. Water was added and the reaction mixture was put into a separatory funnel. The aqueous layer was extracted 5x with CH₂Cl₂. The combined organic layers were dried over anhydrous Na₂SO₄ and filtered. After concentration of the solvent and twice co-distillation with benzene the crude product (747 mg, yellow up to orange resin) was purified twofold by means of CC with CH₂Cl₂ : MeOH = 40 : 1 (Rf: 0.13) as eluent giving 95 mg (0.30 mmol; 10 %) of 2(E)-N-[5-Methyl-3-(pyrrolidin-1-yl)hexa-2,4-dien-1-ylidene]benzenesulfonamide as a yellow resin.
¹H NMR (DMSO-d₆, δ, 400 MHz) : 1.51 (d, *J* = 1.2 Hz, 3H, 6*-H), 1.77 - 1.97 (m, 4H, 2CH₂), 1.88 (d, *J* = 1.4 Hz, 3H, CH₃*), 3.21 - 3.58 (m, 4H, 2NCH₂), 5.30 (d, *J* = 11.2 Hz, 1H, 2-H), 5.91 (t, *J* = 1.5 Hz, 1H, 4-H), 7.47 - 7.57 (m, 3H, ArH), 7.64 - 7.69 (m, 2H, ArH), 8.09 (d, *J* = 11.2 Hz, 1H, 1-H) ppm. ¹³C NMR (DMSO-d₆, δ, 100 MHz) : 20.20 (C-6*), 24.51, 24.77 (2CH₂), 25.10 (CH₃*), 48.79, 50.23 (2NCH₂), 97.12 (C-2), 117.75 (C-4), 126.24, 129.12, 131.79 (ArC), 142.83 (ArC_{q}), 143.26 (C-5), 165.02 (C-3), 167.09 (C-1) ppm.

### Example 7: (2E)-N-(3-Dimethylamino-5-methylhexa-2,4-dien-1-ylidene)benzenesulfonamide

573 mg (3.76 mmol) of N,N,2,2-tetramethyl-2,3-dihydropyridin-4-amine were co-distilled twice with dry benzene and dissolved in 33 ml of dry CH₂Cl₂. To this solution 1141 mg (11.28 mmol, 1.57 ml) of dry triethylamine and 698 mg (3.95 mmol, 0.50 ml) of benzene sulfonyl chloride were added (yellow solution). The reaction mixture was put under an Argon-atmosphere and was stirred for 4 days at room temperature. Water was added and stirring was continued for 15 min. The reaction mixture was put into a separatory funnel. The organic layer was collected and the aqueous layer was further extracted 4x with CH₂Cl₂. The combined organic layers were dried over anhydrous Na₂SO₄ and filtered. After concentration of the solvent and twice co-distillation with benzene the crude product (1024 mg, dark orange resin) was purified by means of CC with CH₂Cl₂: MeOH = 30: 1 (Rf: 0.23) as eluent. Fractions containing the product and small traces of impurities were collected and crystallized twofold with EtOAc/CH. Fractions containing the product and greater traces of impurities were crystallized threefold with EtOAc/Ac. All in all 175 mg (0.60 mmol; 16 %) of (2E)-N-(3-Dimethylamino-5-methylhexa-2,4-dien-1-ylidene)benzenesulfonamide were obtained as a white, cotton like solid.
m.p.: (EtOAc/CH) = 93°C
¹H NMR (DMSO-d₆, δ, 400 MHz): 1.48 (d, *J* = 1.2 Hz, 3H, 6*-H), 1.89 (d, *J* = 1.5 Hz, 3H, CH₃*), 3.04 (s, 3H, NCH₃), 3.06 (s, 3H, NCH₃), 5.41 (d, *J* = 11.1 Hz, 1H, 2-H), 5.87 (t, *J* = 1.5 Hz, 1H, 4-H), 7.48 - 7.58 (m, 3H, ArH), 7.65 - 7.68 (m, 2H, ArH), 8.10 (d, *J* = 11.1 Hz, 1H, 1-H) ppm. ¹³C NMR (DMSO-d₆, δ, 100 MHz) : 20.10 (C-6*), 24.99 (CH₃*), 39.79, 41.44 (2NCH₃), 96.51 (C-2), 117.34 (C-4), 126.29, 129.14, 131.86 (ArC), 142.65 (ArC_{q}), 143.74 (C-5), 167.81 (C-3), 168.03 (C-1) ppm.

### Example 8: (2E)-4-Methyl-N-[5-methyl-3-(pyrrolidin-1-yl)hexa-2,4-dien-1-ylidene]benzenesulfonamide

610 mg (3.42 mmol) of 2,2-dimethyl-4-(pyrrolidin-1-yl)-2,3-dihydropyridine were co-distilled twice with dry benzene and dissolved in 30 ml of dry CH₂Cl₂. To this solution 1038 mg (10.26 mmol, 1.43 ml) of dry triethylamine and 685 mg (3.59 mmol) of 4-toluene sulfonyl chloride were added. The reaction mixture was put under an Argon-atmosphere and was stirred for 3 days at room temperature. Water was added and stirring was continued for 15 min. The reaction mixture was put into a separatory funnel. The organic layer was collected and the aqueous layer was further extracted 4x with CH₂Cl₂. The combined organic layers were dried over anhydrous Na₂SO₄ and filtered. After concentration of the solvent and twice co-distillation with benzene the crude product (934 mg, orange resin) was purified by means of CC with CH₂Cl₂:MeOH = 30:1 (Rf: 0.27) as eluent giving 260 mg of a yellow resin which was crystallized from EtOAc giving 108 mg (0.33 mmol; 10 %) of (2E)-4-Methyl-N-[5-methyl-3-(pyrrolidin-1-yl)hexa-2,4-dien-1-ylidene]benzenesulfonamide as white needles.
m.p.: (EtOAc) = 117°C
¹H NMR (DMSO-d₆, δ, 400 MHz): 1.51 (d, *J* = 1.2 Hz, 3H, 6*-H), 1.78 - 1.94 (m, 4H, 2CH₂), 1.87 (d, *J* = 1.4 Hz, 3H, CH₃*), 2.33 (s, 3H, ArCH₃), 3.20 - 3.57 (m, 4H, 2NCH₂), 5.27 (d, *J* = 11.2 Hz, 1H, 2-H), 5.90 (t, *J* = 1.4 Hz, 1H, 4-H), 7.31 (d, *J* = 7.7 Hz, 2H, ArH), 7.55 (d, *J* = 8.2 Hz, 2H, ArH), 8.07 (d, *J* = 11.2 Hz, 1H, 1-H) ppm. ¹³C NMR (DMSO-d₆, δ, 100 MHz): 20.17 (C-6*), 21.08 (ArCH₃), 24.50, 24.76 (2CH₂), 25.05 (CH₃*), 48.71, 50.14 (2NCH₂), 96.87 (C-2), 117.77 (C-4), 126.32, 129.50 (ArC), 139.89, 141.89 (ArC_{q}), 143.12 (C-5), 164.75 (C-3), 166.93 (C-1) ppm.

### Example 9: (2E)-N-[5-Methyl-3-(azepan-1-yl)hexa-2,4-dien-1-ylidene]benzenesulfonamide

730 mg (3.54 mmol) of 1-(2,2-dimethyl-2,3-dihydropyridin-4-yl)azepane were co-distilled twice with benzene and then dissolved in CH₂Cl₂. Afterwards, 657 mg (3.72 mmol) of benzene sulfonyl chloride and 1074 mg (10.61 mmol) triethylamine were added. The reaction mixture was set under Argon and was stirred for one day at room temperature. Then water was added and stirring was continued for 15 minutes. The aqueous layer was extracted 4 times with CH₂Cl₂. The combined organic layers were dried over anhydrous Na₂SO₄ and filtered. The solvent was evaporated *in vacuo* and the product (1158 mg, red resin) was purified by means of CC with CH₂Cl₂: MeOH = 30:1 (Rf: 0.32) as eluent. Fractions containing the product (906 mg, orange to yellow resin) were collected and crystallized twofold with EtOAc/CH giving 58 mg (0.16 mmol 5%) of (2E)-N-[5-Methyl-3-(azepan-1-yl)hexa-2,4-dien-1-ylidene]benzenesulfonamide as white needles.
m.p. (EtOAc/Cyclohexane): 132°C
¹H NMR (DMSO-d₆, δ, 400 MHz): 1.35 - 1.76 (m, 8H, 2'-H, 3'-H), 1.48 (s, 3H, 6*-H), 1.88 (s, 3H, CH₃*), 3.39 - 3.64 (m, 4H, 1'-H), 5.46 (d, *J* = 11.0 Hz, 1H, 2-H), 5.92 (s, 1H, 4-H), 7.49 - 7.68 (m, 5H, ArH), 8.10 (d, *J* = 11.0 Hz, 1H, 1-H) ppm. ¹³C NMR (DMSO-d₆, δ, 100 MHz) : 20.25 (C-6*), 24.98 (CH₃*), 25.28, 25.55, 26.44, 28.45 (C-2', C-3'), 50.22, 52.12 (C-1'), 96.11 (C-2), 117.16 (C-4), 126.37, 129.19, 131.93 (ArC), 142.53 (ArC_{q}), 143.41 (C-5), 167.17 (C-3), 168.47 (C-1) ppm.

### Example 10: (2E)-N-[5-Methyl-3-(piperidin-1-yl)hexa-2,4-dien-1-ylidene]methanesulfonamide

862 mg (4.48 mmol) of 2,2-dimethyl-4-(piperidin-1-yl)-2,3-dihydropyridine were co-distilled twice with dry benzene and dissolved in 39 ml of dry CH₂Cl₂. To this solution 1.36 g (13.44 mmol, 1.87 ml) of dry triethylamine and 539 mg (4.70 mmol, 364 µl) of methane sulfonyl chloride were added. The reaction mixture was put under Argon and was stirred overnight at room temperature. Water was added and stirring was continued for 15 min. The reaction mixture was put into a separatory funnel. The organic layer was collected and the aqueous layer was further extracted 4x with CH₂Cl₂. The combined organic layers were dried over anhydrous Na₂SO₄ and filtered. After evaporation of the solvent the crude product (1.084 g, light orange resin) was purified by means of CC with CH₂Cl₂ : MeOH = 70 : 1 over basic aluminum oxide followed by repeated purification on silica gel with CH₂Cl₂ : MeOH = 30 : 1 as 35 mg (0.13 mmol; 3 %) of (2E)-N-[5-Methyl-3-(piperidin-1-yl)hexa-2,4-dien-1-ylidene]methanesulfonamide as yellow resin.
¹H NMR (DMSO-d₆, δ, 400 MHz): 1.40 - 1.67 (m, 6H, 3CH₂), 1.57 (s, 3H, 6*-H), 1.89 (s, 3H, CH₃*), 2.76 (s, 3H, SO₂CH₃), 3.51 (br, s, 4H, 2NCH₂), 5.55 (d, *J* = 10.8 Hz, 1H, 2-H), 5.87 (s, 1H, 4-H), 8.16 (d, *J* = 10.8 Hz, 1H, 1-H) ppm; ¹³C NMR (DMSO-d₆, δ, 100 MHz): 20.12 (C-6*), 23.81 (CH₂), 24.97 (CH₃*), 25.49, 26.59 (2CH₂), 41.45 (SO₂CH₃), 47.68, 50.27 (2NCH₂), 95.31 (C-2), 117.41 (C-4), 143.50 (C-5), 165.50 (C-3), 168.73 (C-1) ppm.

### Example 11: (2E)-N-[3-(Azepan-1-yl)-5-methylhexa-2,4-dien-1-ylidene]methanesulfonamide

741 mg (3.59 mmol) of 1-(2,2-dimethyl-2,3-dihydropyridin-4-yl)azepane were co-distilled twice with dry benzene and dissolved in 38 ml of dry CH₂Cl₂. To this solution 1.09 mg (10.77 mmol, 1.5 ml) of dry triethylamine and 432 mg (3.78 mmol) of methane sulfonyl chloride were added. The reaction mixture was put under an Argon-atmosphere and was it stirred for 2 days at room temperature. After two days, water was added and stirring was continued for 15 minutes. The aqueous layer was extracted 4x with CH₂Cl₂. The combined organic layers were dried over anhydrous Na₂SO₄ and filtered. The solvent was evaporated *in vacuo* and the crude product (702 mg, orange-yellow resin) was purified by means of CC over Alox and CH₂Cl₂ : MeOH = 70 : 1 (Rf: 0.) as eluent. Fractions containing the product (346 mg, brown resin) were collected and it was purified by means of CC over silica gel as stationary phase and CH₂Cl₂ : MeOH = 70 : 1 as eluent. Fractions containing the product were combined and evaporated *in vacuo* giving 149 mg (0.524mmol, 15%) of (2E)-N-[3-(Azepan-1-yl)-5-methyl-hexa-2,4-dien-1-ylidene]methanesulfonamide as a transparent colorless resin.
¹H NMR (CDCl₃, δ, 400 MHz) : 1.47 - 1.73 (m, 6H, 2'-H, 3'-H), 1.68 (d, *J* = 1.3 Hz, 3H, 6*-H), 1.75 - 1.89 (m, 2H, 2'-H, 3'-H), 1.94 ((d, *J* = 1.5 Hz, 3H, CH₃*), 2.93 (s, 3H, SO₂CH₃), 3.46 - 3.58 (m, 4H, 1'-H), 5.52 (d, *J* = 10.8 Hz, 1H, 2-H), 5.73 (t, *J* = 1.4 Hz, 1H, 4-H), 8.37 (d, *J* = 10.8 Hz, 1H, 1-H) ppm; ¹³C NMR (CDCl₃, δ, 100 MHz): 20.39 (C-6*), 25.39 (CH₃*), 25.64, 25.89, 26.92, 29.12 (C-2', C-3'), 41.20 (SO₂CH₃), 50.54, 52.02 (C-1'), 96.42 (C-2), 116.59 (C-4), 144.46 (C-5), 166.45 (C-3), 169.39 (C-1) ppm.

### Example 12: Cytotoxicity assay using L6-cells

Assays were performed in 96-well microtiter plates, each well containing 100 µl of RPMI 1640 medium supplemented with 1% L-glutamine (200 mM) and 10% fetal bovine serum, and 4000 L-6 cell, a primary cell line derived from rat skeletal myoblasts (Page, C. ; et al. J. Oncol. 1993, 3, 473-476; Ahmed, S. A.; Gogal, R. M.; et al. J. Immunol. Methods 1994, 170 211-224).

Serial drug dilutions of eleven 3-fold dilution steps covering a range from 100 to 0.002 µg/ml were prepared. After 70 hours of incubation the plates were inspected under an inverted microscope to assure growth of the controls and sterile conditions. 10 µl of Alamar Blue was then added to each well and the plates incubated for another 2 hours. Then the plates were read with a Spectramax Gemini XS microplate fluorometer (Molecular Devices Cooperation, USA) using an excitation wave length of 536 nm and an emission wave length of 588 nm. The IC₅₀ values were calculated by linear regression (Huber, W. ; et al. Acta Trop. 1993, 55, 257-261.) from the sigmoidal dose inhibition curves using SoftmaxPro software (Molecular Devices Cooperation, USA). Podophyllotoxin (Sigma P4405) was used as control.

**Table 1: Results for cytotoxicity against L-6 cells expressed as IC₅₀ values (µM)**

| Compound | IC₅₀ (µM) |
|---|---|
| **Example 2** | 206 |
| **Example 3** | 250 |

The results show that both investigated sulfonylimines according to the invention have high micro molar IC₅₀ values with respect to the investigated mammal cell line.

### Example 13: Antimicrobial and antifungal activity

A drop plate method assay (Alajlani, M.; et al. Chromatographia 2016, 79, 1527-1532.) with modification was performed to detect the antimicrobial and antifungal activity against the Gram positive bacteria *Arthrobacter aurescens* and the yeast strain *Candida krusei.* All compounds were dissolved in DMSO to a concentration of 1 mg/ml. Using sterile micropipette 10 µl of each compound was directly but gently dropped over seeded agar plate with test organism. The liquid was allowed to diffuse before the plate was inverted and incubated. The growth conditions for every strain was considered. The results were noted when a lawn of the indicator organism appeared on the plate (approximately 10-16 h).

The magnitude of activity was noted as high activity (++), Medium activity (+),and no activity(-).

**Table 2: Antimicrobial and antifungal activities of compounds were detected over the test organisms.**

| Compounds | *Arthrobacter aurescens* DSM20116 | *Candida krusei* CCMM L10 |
|---|---|---|
| **Example 3** | ++ | + |
| **Example 5** | ++ | + |
| **Example 6** | ++ | + |
| **Example 7** | ++ | + |
| **Example 8** | ++ | + |
| **Example 10** | ++ | + |

The investigated sulfonylimines inhibited the growth of the gram-positive bacteria Arthobacter at the investigated concentration. Moreover, these compounds showed an activity against the pathogen yeast strain Candida krusei. Based on the previous results, no cytotoxic effect is expected at the investigated concentration (1 mg/ml corresponds to a molar concentration of about 2.5 µmol/ml for Example 3, whereas the IC₅₀ for cytotoxicity (see above) was 100 times higher).

## Claims

1. A sulfonylimine of general formula (I) or a salt thereof 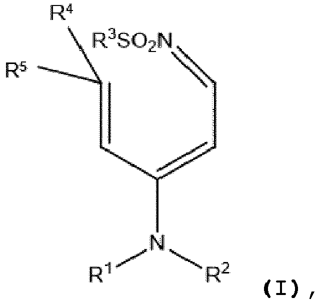 wherein
R¹ and R² are independently from each other selected from the group consisting of hydrogen and a substituted or unsubstituted C₁-C₁₀-alkyl group, or
R¹ and R² form together with the nitrogen to which they are bound a substituted or unsubstituted 5-, 6- or 7-membered heterocyclyl group,
R³ is selected from the group consisting of
a) a substituted or unsubstituted C₅-C₁₄-aryl group,
b) a substituted or unsubstituted C₁-C₁₀-alkyl group,
c) a substituted or unsubstituted C₅-C₁₄-aryl-C₁-C₄-alkyl group,
d) a substituted or unsubstituted heteroaryl or a heteroaryl-C₁-C₄-alkyl group, wherein the heteroaryl group comprises a 5 to 14 membered aromatic ring system with at least one heteroatom selected from the group consisting of N, O, and S atoms,
e) a substituted or unsubstituted heterocyclyl or a heterocyclyl-C₁-C₄-alkyl group, wherein the heterocyclyl group comprises a 5 to 14 membered non-aromatic ring system with at least one heteroatom selected from the group consisting of O and S atoms,
f) a substituted or unsubstituted C₂-C₁₀-alkenyl group containing one or more double bonds,
g) a substituted or unsubstituted C₂-C₁₀-alkinyl group containing one or more triple bonds,
h) a substituted or unsubstituted C₅-C₁₄-aryl-C₂-C₄-alkenyl group containing one or more double bonds,
i) a substituted or unsubstituted C₅-C₁₄-aryl-C₂-C₄-alkinyl group containing one or more triple bonds,
j) a substituted or unsubstituted heteroaryl-C₂-C₄-alkenyl group containing one or more double bonds, and
k) a substituted or unsubstituted heteroaryl-C₂-C₄-alkinyl group containing one or more triple bonds, wherein the heteroaryl is 5 to 14 membered aromatic ring system with at least one heteroatom selected from the group consisting of N, O, and S
l) a substituted or unsubstituted heterocyclyl-C₂-C₄-alkenyl group containing one or more double bonds, and
m) a substituted or unsubstituted heterocyclyl-C₂-C₄-alkinyl group containing one or more triple bonds, wherein the heterocyclyl is 5 to 14 membered non-aromatic ring system with at least one heteroatom selected from the group consisting of O and S,
and R⁴ and R⁵ both are independently from each other a substituted or unsubstituted C₁-C₁₀-alkyl group or R⁴ is a substituted or unsubstituted C₁-C₁₀-alkyl group and R⁵ is hydrogen.

2. A process for preparing a sulfonylimine comprising a step of reacting a compound of general formula (II) 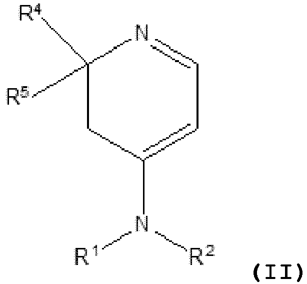 with a sulfonyl halide of general formula (III) 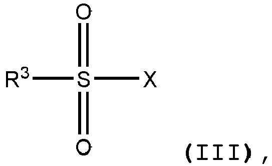 to obtain a sulfonylimine of general formula (I) 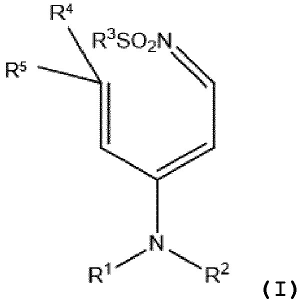 or a salt thereof,
wherein
R¹ and R² are independently from each other selected from the group consisting of hydrogen and a substituted or unsubstituted C₁-C₁₀-alkyl group, or
R¹ and R² form together with the nitrogen to which they are bound a substituted or unsubstituted 5-, 6- or 7-membered heterocyclyl group,
R³ is selected from the group consisting of
a) a substituted or unsubstituted C₅-C₁₄-aryl group,
b) a substituted or unsubstituted C₁-C₁₀-alkyl group,
c) a substituted or unsubstituted C₅-C₁₄-aryl-C₁-C₄-alkyl group,
d) a substituted or unsubstituted heteroaryl or a heteroaryl-C₁-C₄-alkyl group, wherein the heteroaryl group comprises a 5 to 14 membered aromatic ring system with at least one heteroatom selected from the group consisting of N, O, and S atoms,
e) a substituted or unsubstituted heterocyclyl or a heterocyclyl-C₁-C₄-alkyl group, wherein the heterocyclyl group comprises a 5 to 14 membered non-aromatic ring system with at least one heteroatom selected from the group consisting of O and S atoms,
f) a substituted or unsubstituted C₂-C₁₀-alkenyl group containing one or more double bonds,
g) a substituted or unsubstituted C₂-C₁₀-alkinyl group containing one or more triple bonds,
h) a substituted or unsubstituted C₅-C₁₄-aryl-C₂-C₄-alkenyl group containing one or more double bonds,
i) a substituted or unsubstituted C₅-C₁₄-aryl-C₂-C₄-alkinyl group containing one or more triple bonds,
j) a substituted or unsubstituted heteroaryl-C₂-C₄-alkenyl group containing one or more double bonds, and
k) a substituted or unsubstituted heteroaryl-C₂-C₄-alkinyl group containing one or more triple bonds, wherein the heteroaryl is 5 to 14 membered aromatic ring system with at least one heteroatom selected from the group consisting of N, O, and S,
l) a substituted or unsubstituted heterocyclyl-C₂-C₄-alkenyl group containing one or more double bonds, and
m) a substituted or unsubstituted heterocyclyl-C₂-C₄-alkinyl group containing one or more triple bonds, wherein the heterocyclyl is 5 to 14 membered non-aromatic ring system with at least one heteroatom selected from the group consisting of O and S,
R⁴ and R⁵ both are independently from each other a substituted or unsubstituted C₁-C₁₀-alkyl group or R⁴ is a substituted or unsubstituted C₁-C₁₀-alkyl group and R⁵ is hydrogen,
and wherein X is a halide selected from the group consisting of F, Cl, and Br.

3. Sulfonylimine according to claim 1 or process according to claim 2, wherein R¹ and R² together with the nitrogen to which they are bound form a heterocyclyl group selected from the group consisting of azepanyl, piperidinyl and pyrrolidinyl.

4. Sulfonylimine according to claim 1, or process according to claim 2, wherein R¹ and R² are independently from each other selected from the group consisting of hydrogen, methyl, and ethyl, preferably methyl.

5. Sulfonylimine according to any one of claims 1 or 3 to 4 or process according to any one of claims 2 to 4, wherein R¹ and R² are identical to each other.

6. Sulfonylimine according to claim 1 or 3 to 5 or process according to any one of claims 2 to 5, wherein R³ is selected from the group of substituted or substituted C₆-C₁₄-aryl and C₁-C₁₀-alkyl such as methyl.

7. Sulfonylimine according to claim 1 or 3 to 6 or process according to any one of claims 2 to 6, wherein R³ is a C₆-aryl, in particular a C₆-aryl substituted in ring position 4, preferably selected from the group of 4-nitrophenyl, 4-chlorophenyl, 4-methylphenyl and phenyl.

8. Sulfonylimine according to claim 1 or 3 to 7 or process according to any one of claims 2 to 7, wherein R⁴ and R⁵ both are methyl.

9. Sulfonylimine according to claim 1 or 3 to 8 or process according to any one of claims 2 to 8, in the form of a salt thereof, wherein the cation is a protonated form of the compound of formula (I) and the anion is a halide anion selected from the group consisting of F⁻, Cl⁻, and Br⁻.

10. Sulfonylimine according to claim 1 or 3 to 9 or process according to any one of claims 2 to 9, wherein the sulfonylimine of formula (I) is selected from the group consisting of
(2E)-N-[5-methyl-3-(piperidin-1-yl)hexa-2,4-dien-1-ylidene]-4-nitrobenzenesulfonamide,
(2E)-N-[5-methyl-3-(pyrrolidin-1-yl)hexa-2,4-dien-1-ylidene]-4-nitrobenzenesulfonamide,
(2E)-N-[5-methyl-3-(piperidin-1-yl)hexa-2,4-dien-1-ylidene]benzenesulfonamide,
(2E)-4-chloro-N-[5-methyl-3-(piperidin-1-yl)hexa-2,4-dien-1-ylidene]benzenesulfonamide,
(2(E)-N-[5-methyl-3-(pyrrolidin-1-yl)hexa-2,4-dien-1-ylidene]benzenesulfonamide,
(2E)-N-(3-dimethylamino-5-methylhexa-2,4-dien-1-ylidene)benzenesulfonamide,
(2E)-4-methyl-N-[5-methyl-3-(pyrrolidin-1-yl)hexa-2,4-dien-1-ylidene]benzenesulfonamide,
(2E)-N-[5-methyl-3-(azepan-1-yl)hexa-2,4-dien-1-ylidene]benzenesulfonamide,
(2E)-N-[5-methyl-3-(piperidin-1-yl)hexa-2,4-dien-1-ylidene]methanesulfonamide,
(2E)-N-[3-(azepan-1-yl)-5-methylhexa-2,4-dien-1-ylidene]methanesulfonamide,
and salts thereof.

11. Process according to any one of claims 2 to 10, wherein the step of reacting the compound of general formula (II) with the sulfonyl halide of general formula (III) is performed in an aprotic solvent, preferably an aprotic polar solvent.

12. Process according to any one of claims 2 to 11, wherein the step of reacting the compound of general formula (II) with the sulfonyl halide of general formula (III) is performed in the presence of a molar excess of an organic base.

13. Process according to any one of claims 2 to 12, further comprising at least one subsequent step of purification such as filtration, liquid-liquid separation, drying, column chromatography, and crystallization.

14. Sulfonylimine according to any one of claims 1 or 3 to 9 for use as medicament.

15. Sulfonylimine according to any one of claims 1 or 3 to 9 for use in the treatment or prevention of an infection, preferably for use in treatment and prevention of a fungal or bacterial infection.
